# EUROPEAN PATENT SPECIFICATION

(11) **EP 2 307 040 B1**
(45) Date of publication and mention of the grant of the patent: **09.09.2015**
(21) Application number: 09755128.7
(22) Date of filing: 18.05.2009
(51) Int. Cl.: A61K 38/09, A61P 31/18, A61P 37/04, A61P 5/08, A61K 45/06

(54) **USE OF GNRH AND SEX HORMONES FOR TREATING VIRAL DISEASE, IN PARTICULAR HIV/AIDS**
GNRH UND SEXHORMONE ZUR BEHANDLUNG VON VIRUSERKRANKUNGEN, INSBESONDERE HIV/AIDS
UTILISATION DE GNRH ET D'HORMONES SEXUELLES POUR LE TRAITEMENT D'UNE MALADIE VIRALE, EN PARTICULIER LE VIH/SIDA

(30) Priority: 29.05.2008 SE 0801255
(43) Date of publication of application: 13.04.2011
(73) Proprietor: Isr Immune System Regulation Ab, 17176 Stockholm (SE)
(72) Inventor: LJUNGBLAD, Ulf, S-Göteborg 412 57 (SE); BACKSTRÖM LUNDGREN, Yen, S-42679 Västra Frölunda (SE); WINQVIST, Ola, S-75653 Uppsala (SE)
(74) Representative: Boult Wade Tennant
(86) International application number: PCT/SE2009/000255
(87) International publication number: WO 2009/145690

(56) References cited:
- WO-A1-96/03138
- WO-A1-99/26630
- WO-A1-02/102401
- WO-A1-2004/096259
- US-A- 4 866 160
- US-A- 5 985 834
- US-A1- 2004 013 641
- US-A1- 2004 138 138
- US-A1- 2007 274 946
- JACOBSON ET AL: "Gonadotropin-releasing hormone increases CD4<+> T-lymphocyte numbers in an animal model of immunodeficiency", JOURNAL OF ALLERGY AND CLINICAL IMMUNOLOGY, MOSBY, INC, US, vol. 104, no. 3, 1 September 1999 (1999-09-01), pages 653-658, XP005687679, ISSN: 0091-6749, DOI: 10.1016/S0091-6749(99)70338-6

## Description

### FIELD OF THE INVENTION

The present invention relates to a method and a means for treating viral disease, in particular HIV/AIDS.

### BACKGROUND OF THE INVENTION

CD4+ T cells are key regulators of immunological responses and have a crucial role in maintaining immune competence. These cells are also the primary target for HIV. There is a great and still unsatisfactorily met need for methods and means of maintaining or even increasing their immune competence.

The decapeptide GnRH (gonadotropin releasing hormone, GnRH1 (in the following: "GnRH"); old name: LHRH), responsible for release of FSH and LH from the anterior pituitary gland, is normally released from the hypothalamus in a pulsative manner. It stimulates the receptors of the anterior pituary gland to secretion of FSH and LH according to the negative feed-back hormonal system (1). Hence castration of a human being leads to increased secretion of GnRH due to the hormonal feed-back system.

High unphysiological levels of GnRH induce an immediate increase of FSH and LH secretion, soon followed by inhibition of FSH and LH secretion. This is due to the fact that high levels of GnRH have an inhibitory effect on the receptors of the anterior pituary gland. Continuous administration of GnRH at high unphysiological levels induces pharmacological castration;
this effect is used in pre-operative treatment of big uterine myomas and in the treatment of endometriosis.

It have been reported that GnRH not only exhibits hormonal effects but also stimulates the immune system (2). McClean and McC luggage (4) observed massive infiltration of small mature lymphocytes in uterine leiomyomas after preoperative treatment with a GnRH agonist. Bardsley et al. (5) made the same observation, indicating a stimulatory effect of GnRH on the immune system. Kerr-Layton et. al. (6) reported chronic plasma cell endometritis in hysterectomy specimens from HIV-infected women in a retrospective analysis. Arver et al. (7) found elevated levels of FSH and LH (hypergonadotropic) in HIV-infected men as did Brockmeyer et al (8). By administering GnRH to diabetes-prone BB rats exhibiting an AIDS-like lymphocyte profile Jacobsson et al. (3) increased CD4 T-lymphocyte numbers.

US 4,866,160 A discloses that LHRH antagonists can be used to treat patients suffering from AIDS; the LHRH antagonists are said to rejuvenate the thymus to make it produce T cells that replace the T cells destroyed by the virus. US 985,836 A discloses novel LHRH antagonists suitable for treating AIDS based on their in-vitro effect on T4 lymphocytes.

US2004/0013641 A1 describes methods for preventing disease, for example HIV, involving administration of LHRH or analogs thereof. This document describes the administration of LHRH so as to disrupt sex-steroid mediated signalling to the thymus thereby stimulating production of T cells by this organ.

### OBJECTS OF THE INVENTION

An object of the present invention is to provide a method of maintaining or even increasing the immune competence of CD4+ T cells in a patient suffering from viral disease, in particular HIV.

Another object of the present invention is the treatment of viral disease in a patient, in particular of HIV/AIDS.

Still another object of the present invention is to provide a pharmaceutical composition or a pharmaceutical composition for treating viral disease in a patient, in particular HIV/AIDS.

An additional object of the present invention is to provide a method use and of manufacture for the composition.

Further objects of the invention will become evident from the following summary of the invention, the description of a number of preferred embodiments illustrated in a drawing, and the appended claims.

### SUMMARY OF THE INVENTION

The present invention provides GnRH or GnRH analog including pharmaceutically acceptable salts thereof for use in a method of treating viral disease wherein the method comprises the concomitant administration of GnRH or GnRH analog including pharmaceutically acceptable salts thereof in an amount sufficient to maintain in the patient an elevated unphysiological plasma level of GnRH or GnRH analog, and of one or several natural, semi-synthetic or synthetic sexual hormones in an amount sufficient to compensate for the castration effect of GnRH or GnRH analog.

The present invention relates to a method of maintaining or increasing the immune competence in T-cell deficient patients, in particular patients suffering from AIDS. The method comprises concomitant administration of an unphysiological amount of GnRH or a GnRH analog and of one or several natural, semi-synthetic of synthetic sexual hormones. The method eliminates the negative effects of chemical castration caused by administration of GnRH or a GnRH analog in an unphysiological amount while not affecting the immune system stimulating effect thereof.

In an adult male person the natural, semi-synthetic or synthetic sexual hormone of the invention is testosterone or an agent having a corresponding hormonal effect.

In an adult female person, the natural, semi-synthetic or synthetic sexual hormone is oestradiol or an agent having a corresponding hormonal effect, in particular in combination with a progestagen. The progestagen is added to avoid the development of endiometrial cancer in the female and to avoid vaginal bleedings. Hysterectomized women do however not benefit by added progestagen.

Concomitant administration of GnRH or a GnRH analog in an unphysiological, in particular a castrating, amount and of the natural, semi-synthetic or synthetic sexual hormone in a castration-compensating amount can extend, for instance, over one or several periods interrupted by administration-free intervals or be continuous. A preferred administration period is from one to two weeks, in particular from 10 days to 14 days. A preferred administration-free interval is from one month to three months.

GnRH analogs are known in the art. A GnRH analog is an agent that mimicks the action of GnRH on the receptors of the anterior pituary gland when administered to a mammal including man. Whereas administration of a GnRH analog in a single low physiological dose or in single low physiological doses spaced in time does stimulate the receptors of the anterior pituary gland and thus acts as a receptor agonist, the continuous administration of a GnRH analog in a high unphysiological dose per time unit will, after initial stimulation of the receptors of the anterior pituary gland, inhibit the secretion of FSH and LH, and thus acts as a receptor antagonist.

Inhibition of FSH and LH secretion induces pharmacological castration. In this application, an unphysiological dose of GnRH' or GnRH analog is a dose resulting in an unphysiological plasma level of GnRH or GnRH, respectively, in particular a castrating plasma level. In this application, an unphysiological plasma level of GnRH or GnRH analog is a level not comprised by range of levels of GnRH normally present or, in case of GnRH analog, a level not comprised in regard of physiological effect by the normal physiological effect range of GnRH in a healthy person.

More particularly, in this application, an unphysiological plasma level of GnRH is a level increased, in particular increased for an extended period of time such as for more than a week or more than a month, in respect of the normal physiological plasma level of GnRH. Also, more particularly, an unphysiological plasma level of GnRH analog is an increased plasma level of GnRH analog not comprised in respect of physiological effect by the normal physiological effect range GnRH in a healthy person, in particular not for an extended period of time such as for more than a week or more than a month.

Any useful form of administration of the GnRH or GnRH analog of the invention including their pharmaceutically acceptable salts is comprised by the invention, in particular intravenous, intramuscular, subcutaneous, sublingual, and nasal administration. Particular preferred are depot and slow or sustained release compositions.

According to another preferred aspect of the invention the viral disease is HIV/AIDS.

It is preferred that the period of administration of the GnRH or GnRH analog substantially overlaps the period of hormonal substitution, such as by more than 50 per cent, preferably by more than 85 per cent, even more preferred by more than 90 or 95 per cent. This combined administration allows to protect the patient from serious side effects of castration such as decreased libido, hot flushes, increased perspiration, increased hear rate, etc. In a male patient hormonal substitution is accomplished by administration of testosterone or an agent having a corresponding hormonal effect, such as a testosterone analog. In this application testosterone analogs are agents, in particular synthetic or semi-synthetic agents, that mimick the hormonal effect of testosterone. Preferred testosterone analogs comprise methyltestosterone and stanozolol. In a female patient hormonal substitution is preferably by administration of a naturally occurring oestrogen, preferably oestradiol or a semi-synthetic ester of oestradiol, or a synthetic or semi-synthetic oestrogen analog. In this application synthetic or semi-synthetic oestrogen analogs are agents that mimic the hormonal effect of oestradiol. Preferred oestrogen analogs comprise conjugated oestrogens, ethynylestradiol and mestranol as well as non-steroidal oestrogens such as dinestrol and diethylstilbestrol.

In a female patient hormonal substitution by a naturally occurring oestrogen or an agent having a corresponding hormonal effect, such as a synthetic or semi-synthetic oestrogen analog; according to an important aspect of the invention this administration is preferably combined with concomitant administration of a progestogen, in particular progesterone, a derivative or analog thereof such as hydroxyprogesterone caproate, medroxyprogesterone acetate, norethisterone acetate, megestrol acetate, medrogestone and norgestrel; this combined administration preferably overlaps by more than 50 per cent, preferably by more than 85 per cent, even more preferred by more than 90 per cent. It is preferred for the progestogen to be administered in combination with the oestrogen, the semi-synthetic ester of oestradiol or estriol, or the synthetic or semi-synthetic oestrogen analog continuously or over periods of from about 10 to 14 days in intervals of from about one to three months.

According to another preferred aspect of the invention the viral disease is selected from: Adenovirus Infection, Alphavirus Infection, Arbovirus Encephalitis, Borna Disease, Bunyavirus Infection, Calicivirus Infection, Chickenpox, Condyloma Acuminata, Coronavirus Infection, Coxsackievirus Infection, Cytomegalovirus Infection, Dengue fever, Contageous Ecthyma, Epstein-Barr Virus Infection, Erythema Infectiosum, Hantavirus Infection, Viral Hemorrhagic Fever, Viral Hepatitis, Herpes Simplex, Herpes Zoster, Infectious Mononucleosis, Influenza, Lassa Fever, Measles, Molluscum Contagiosum, Mumps, Paramyxovirus Infection, Phlebotomus Fever, Polyomavirus Infection, Rabies, Respiratory Syncytial Virus Infection, Rift Valley Fever, Rubella, Slow Virus Diseases, Smallpox, Subacute Sclerosing Panencephalitis, Tumor Virus Infections, West Nile Fever, and Yellow Fever.

The GnRH analogs of the invention comprise preferably at least four peptide bonds, more preferred at least seven peptide bonds.

GnRH analogs useful in the invention comprise but are not restricted to:

| Structure | Generic Name |
|---|---|
| D-Trp⁶Pro⁹NHEt GnRH | deslorelin |
| [DesGly¹⁰, D-2-Methyl-Trp⁶, Pro-NHEt] GnRH | avorelin |
| D-Leu⁶, des-Gly-NH₂¹⁰] GnRH (1-9) NHEt | leuprolide |
| D-trp⁶ GnRH | triptorelin |
| [D-Ser(but)⁶, des-Gly-NH₂¹⁰] GnRH (1-9) NHEt | buserelin |
| Des-Gly¹⁰-NH₂ GnRH ethylamide | fertirelin |
| [D-Trp⁶, MeLeu⁷, des-Gly-NH₂¹⁰] GnRH (1-9) NHEt | lutrelin |
| [D-Ser (Bu^{t})⁶, Azgly¹⁰] GnRH | goserelin |
| [D-His (benzyl)⁶, des-Gly-NH₂¹⁰] GNRH (1-9) NHEt | historelin |
| [3-(2-naphthyl)⁶, D-Ala⁷] GnRH (1-9) NHEt | nafarelin |

Most preferred are triptorelin, nafarelin, buserelin, goserelin, and leuprolide. A preferred formulation of triptorelin is marketed under the trade name Decapetyl® Depot (triptorelin acetate). A preferred formulation of goserelin is marked under the trade name Zoladex®. Also preferred is natural GnRH.

A liquid composition for the controlled release of GnRH or GnRH analog, such as nafarelin and neuprolide, is disclosed in U.S. Patent No. 6.051,558.

According to a preferred aspect of the invention GnRH or GnRH analog is comprised by a suitable pharmaceutical composition, in particular a slow release or depot composition, such as Zoladex® comprising goserelin acetate which, when injected subcutaneously in a dose equivalent of 3.6 mg goserelin into the anterior abdominal wall, provides effective suppression of oestradiol or testosterone for 28 days. An alternative treatment with subcutaneous Zoladex® is 10.8 mg every 12^{th} week. Similarly, intramuscular injection of Decapeptyl-Depot® (triptorelin pamoate) 3.75 mg or 11.25 mg provides effective suppression of FSH and LH release during four and twelve weeks, respectively.

According a particularly important aspect of the invention is disclosed a composition for use in the treatment of viral disease comprising GnRH or a GnRH analog, one or several natural, semi-synthetic or synthetic sexual hormones, and a pharmaceutically acceptable carrier, wherein the GnRH or GnRH analog is present in an amount sufficient to maintain in a patient an elevated unphysiological plasma level of GnRH or GnRH analog and wherein the one or several natural, semi-synthetic or synthetic sexual hormones is/are present in an amount sufficient to compensate for the castration effect of GnRH or GnRH analog.

The composition of the aforementioned kind is designed for treatment of an adult male person comprises an unphysiological amount of GnRH or a GnRH analog, a castration attenuating or eliminating amount of testosterone or an agent exhibiting a corresponding hormonal effect, and optionally a pharmaceutically acceptable carrier.

Alternatively the composition of the aforementioned kind is designed for treatment of an adult female person comprises an unphysiological amount of GnRH or a GnRH analog, a castration attenuating or eliminating amount of oestradiol or an agent exhibiting a corresponding hormonal effect, optionally a progestagen, and/or a pharmaceutically acceptable carrier.

It is preferred for the pharmaceutical composition of the invention to be a depot or slow or sustained release composition. It is also preferred for the pharmaceutical composition of the invention to comprise a bioerodible matrix.

The patient in need of stimulation of his or her immune system or of maintaining the immune system in an adequate state is preferably treated with a dose of GnRH or GnRH analog sufficient to substantially suppress the release of FSH- and LH over an extended period of time, such as for one month or longer. Substantial suppression comprises suppression by more than 80 %, more preferred by more than 90 %, even more preferred by more than 95 %, most preferred by more than 98 and even 99 %. The patient thus is preferably treated with a dose of GnRH or GNRH analog sufficient for chemical castration. Negative aspects of castration are compensated for as disclosed above.

According to the present invention is also disclosed the use of the pharmaceutical composition of the invention for the treatment of viral disease, in particular HIV/AIDS.

The invention will now be explained in greater detail by reference to preferred but not limiting embodiments illustrated in a number of figures.

### SHORT DESCRIPTION OF THE FIGURES

In the Figures "GnRH" refers to the GnRH analog leuprolide acetate.
- Fig. 1: Effect of IL-2 and IL-2 + GNRH analog on PBMC proliferation in PBMC from a healthy donor;
- Fig. 2: Effect of IL-2 and IL-2 + GnRH analog on the expression of the IL-2 receptor α chain (CD25) on CD4+ and CD8+ T cells from healthy donors;
- Figs. 3a and 3b: Effect of GnRH analog on the expression of CD25 on CD4+ T cells;
- Figs. 4a and 4b: Effect of GnRH analog on the expression of HLA ABC (Human Leucocyte Antigen-A, -B, -C) on CD4+ T cells;
- Figs. 5a and 5b: Effect of GnRH analog on the expression of CD69 on CD4+ T cells;
- Fig. 6: Effect of IL-2 or IL-2 + GnRH analog on the expression of HLA ABC on T cells from one healthy donor and two HIV-infected patients.

### DESCRIPTION OF PREFERRED EMBODIMENTS

### EXAMPLE 1

*Isolation of peripheral blood mononuclear cells* (*PBMC*). Lymphocytes and monocytes were purified from blood samples from healthy donors using Ficoll-Paque Plus (Amersham Biosciences, Uppsala, Sweden). The blood sample was diluted with PBS and carefully layered on a Ficoll-sodium diatrizoate solution, after which the two-phase system was centrifuged at 400xg for 30 min.

This resulted in the collection of PBMC at the interphase between the Ficoll solution and plasma, whereas erythrocytes and granulocytes gathered at the bottom of the tubes. The lymphocyte layer was collected using a Pasteur pipette, and the cells washed with HBSS to remove excess Ficoll-Paque Plus, plasma and platelets. The cells were counted and dissolved in RPMI medium containing 10 % HuS, 1 % PeSt and 1 % glutamine.

### EXAMPLE 2

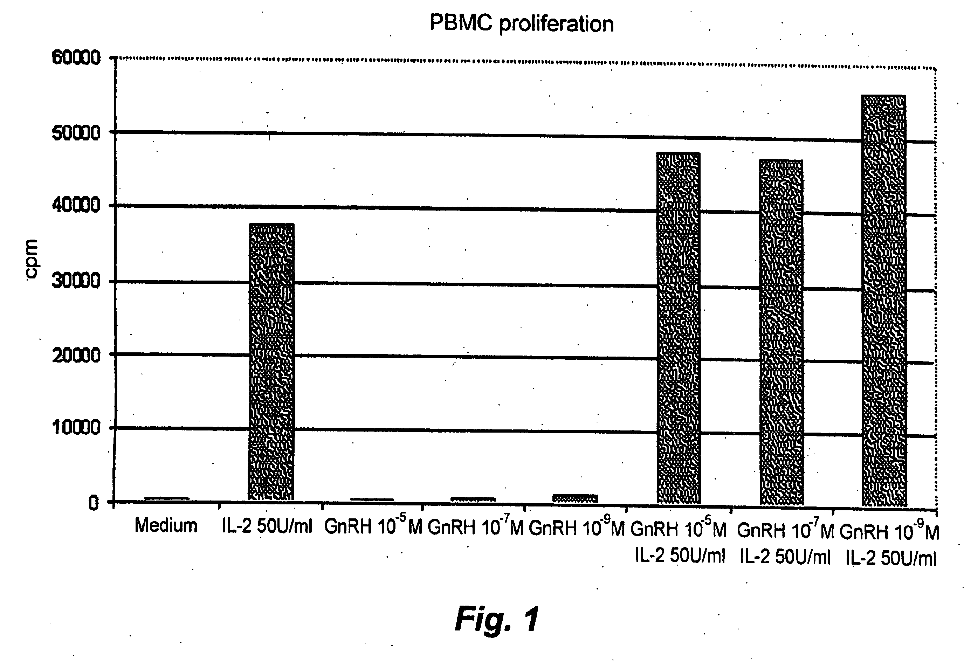

*Proliferation assay*. Interleukin (IL)-2 plays a pivotal role in lymphocyte activation and proliferation. For this reason the effect of GnRH analog on IL-2-induced PBMC proliferation was investigated. PBMCs from a healthy donor were plated in roundbottomed 96-well plates at a concentration of 1x10⁵ cells/100 µL in the culture medium described above. Just after the onset of culture, the cells were treated with IL-2 (Proleukin, Chiron Corporation, Emeryville, Ca, USA) at a concentration of 50U/mL and with three different concentrations between 1x10⁻⁹ and 1x10⁻⁵ M of GnRH analog (Leuprolide acetate, Nordic Drugs, Limhamn, Sweden) or culture medium (Fig. 1). Plates were kept at 37°C with 5% CO₂ for three days before 1 µCi of [³H]thymidine was added to each well and plates were incubated for another 18 h. The well content was then transferred to a glass fiber filter (Wallac, Turku, Finland) by a cell harvester (TOMTEC, Hamden, CT, USA). MeltiLex A - Melt-on scintillator sheets (Wallac, Turku, Finland) were placed on top of the filters and melted at 85°C. Radioactivity was measured using a 1205 Betaplate Liquid ScintillCounter (Wallac, Turku, Finland).

### EXAMPLE 3

### Stimulation of T-cells from healthy donors with GnRH analog.

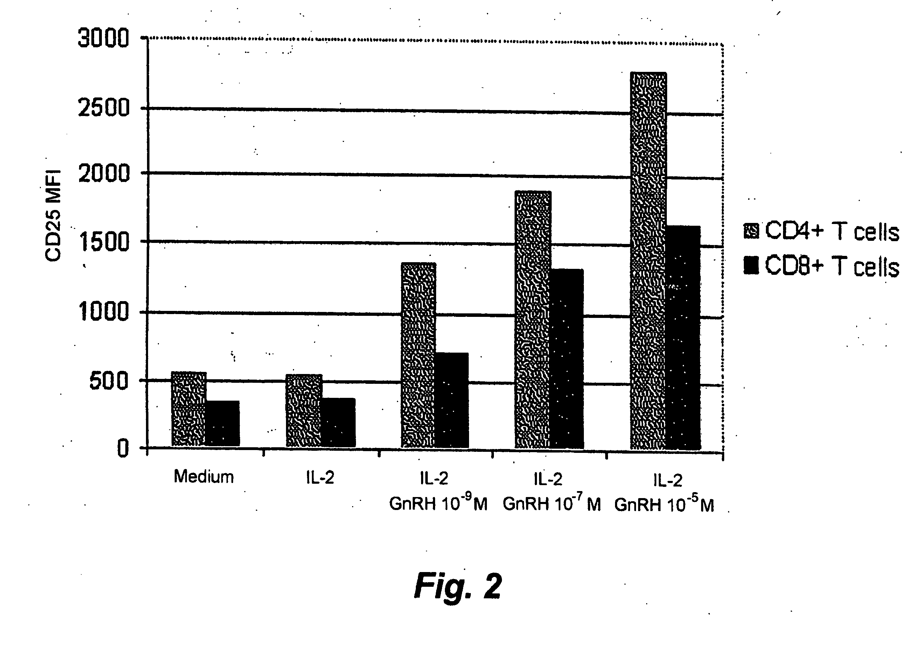

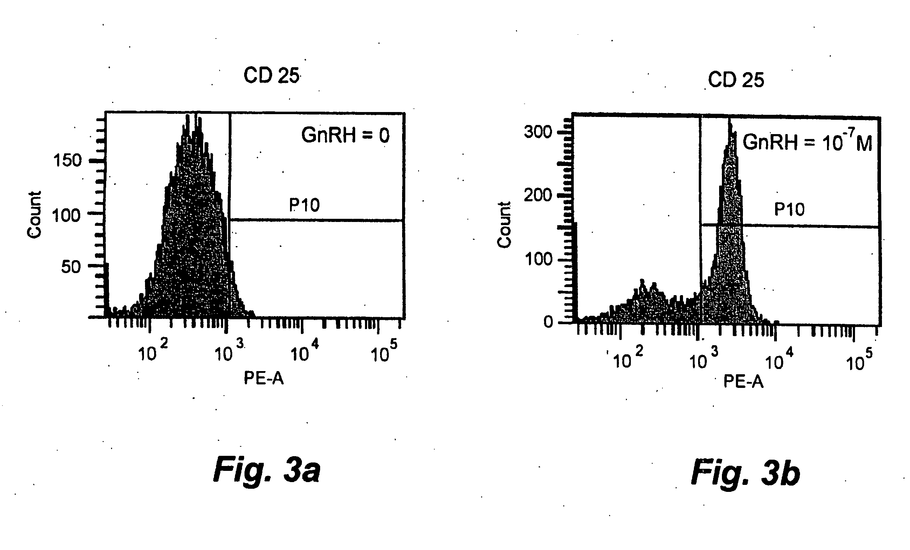

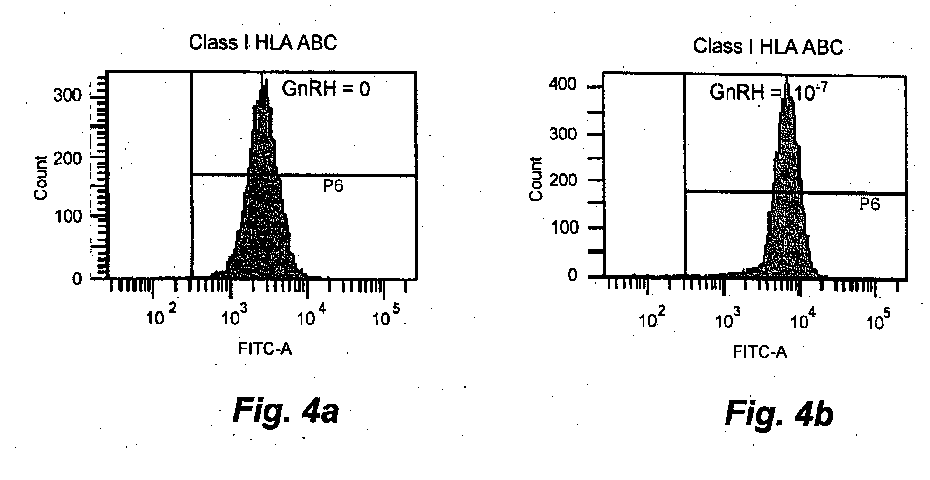

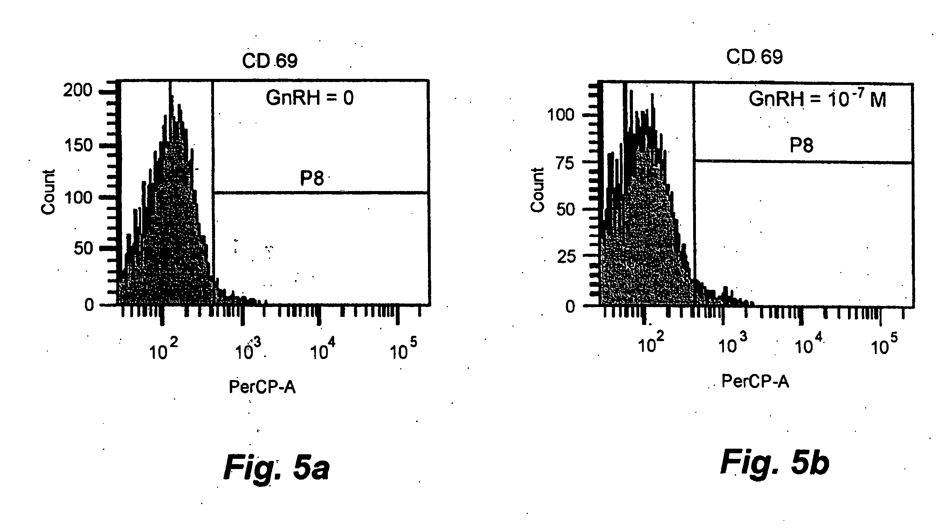

Ficoll-separated PBMCs from healthy donors were cultured in 6-well plates at a concentration of 3x10⁶ cells/well in the culture medium described above. The cells were treated with 50U/mL of IL-2 (Proleukin) and with three different concentrations, 1x10⁻⁹, 1x10⁻⁹, 1x10⁻⁵ M of Leuprolide acetate; in one experiment the cells were treated with culture medium only. The plates were incubated for three days at 37°C with 5% CO₂. After incubation, the cells were washed twice with a buffer assigned for fluorescence activated cell sorting (FACS) containing 0.05% NaN₃, 0.1% bovine serum albumin (BSA) and 0.4% trisodium citrate dihydrate in PBS. The cell suspensions were incubated with fluorochrome-conjugated monoclonal antibodies (mAbs) for 30 minutes at 4°C in the dark. After a final wash, the cells were suspended in 500 µl of the FACS buffer and analysed. Mouse-anti-human mAbs conjugated to fluorescein isothiocyanate (FITC), phycoerythrin (PE), peridinin chlorophyll protein (PerCP), CyChrome, allophycocyanin (APC) or Pacific Blue were used for all antigens. All antibodies used for flow cytometry were purchased from Becton Dickinson (BD) Biosciences/ Pharmingen, San Diego, USA. The effect IL-2 and the combined effect of IL-2 and Leuprolide acetate on the expression of the IL-2 α chain (CD25) on CD4 and CD8 T cells in is shown in Fig. 2. The effect of IL-2 and the combined effect of IL-2 and Leuprolide acetate on the expression of CD25, Class I (HLA ABC) and CD69 on CD4+ T cells is shown in Figs. 3a and 3b, Figs. 4a, 4b and Figs. 5a, 5b, respectively.

### EXAMPLE 4

*Characterization of cells by flow cytometry.* Flow cytometry (FACS) was used to investigate the expression of different surface markers on cell populations of interest. CD4+ and CD8+ T cells were identified by respective marker, and their expression of the activation markers CD25, CD69 and HLA DR was evaluated. The flow cytometry assay was performed on a FACS Aria (Becton Dickinson, Immunocytometry systems, San José, Ca, USA), and for data analyses, BD FACS Diva software was used.

### EXAMPLE 5.

### Stimulation of T-cells from HIV-patients with GnRH-analog.

Because of the risk of contamination, blood samples from HIV-patients had to be handled and analyzed in a closed automated system. Heparinised blood from three HIV-patients and two healthy individuals was diluted with serum-free culture medium (Aim V from Invitrogen) and stimulated with 50 U/mL IL-2. and four different concentrations of the GnRH analog leuprolide acetate between 10⁻¹ and 10⁻⁵ M. The blood samples were handled by a dispensation robot (Prep Plus) and incubated, lysed, stabilized and fixed in a TQ-Prep machine. The incubation time was 18 h and the temperature 37 °C. After the incubation with Leuprolide acetate and IL-2, the cells were stained with fluorochrome-conjugated mAbs for 10 min, and red blood cells were lysed. The lymphocytes were subsequently analysed by flow cytometry on an Epics XL-MCL (all machines from Beckman Coulter). T-cells were identified by their forward-and side scatter properties, and the surface expression of CD3. The expression of the activation markers CD25, CD69, HLA ABC, and HLA DR on T cells was evaluated.

### EXAMPLE 6

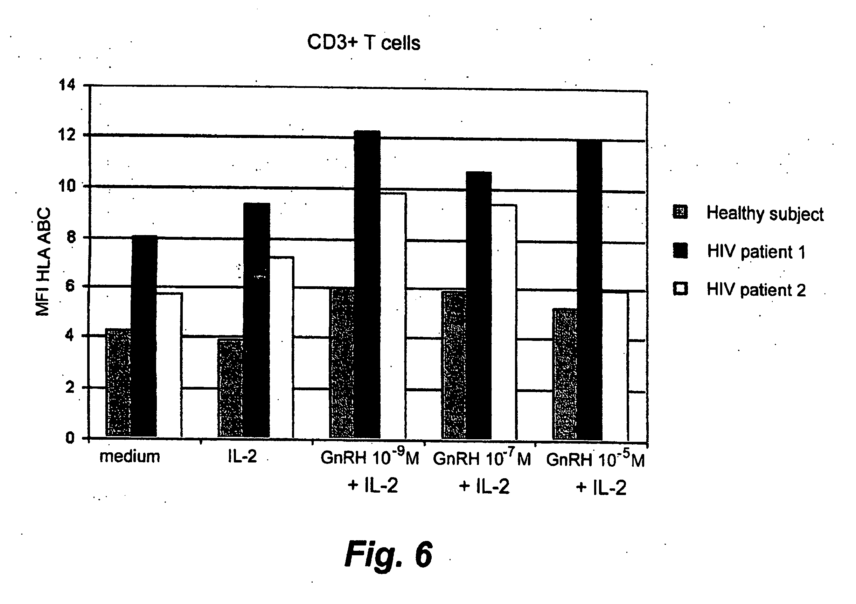

*Proliferation*. Treatment of a PBMC (Peripheral Blood Mononuclear Cell] sample with GnRH and IL-2 resulted in increased proliferation compared with cells treated with IL-2 alone and untreated controls (Fig. 1). Incubation of whole blood from healthy subjects or HIV-infected patients with GnRH for 18 hours caused upregulation of HLA ABC on T cells (Fig. 6). The expression of HLA DR was increased in one of three HIV-patients, and only a slight increase in the expression of CD69 and CD25 was seen in HIV-infected patients and control subjects, possibly because of the short incubation time.

### EXAMPLE 7

*Exemplary treatment of an adult male AIDS patient*. The patient is administered (a) Decapeptyl-Depot® (triptorelin pamoate) 3.75 mg by intramuscular injection in intervals of one month; (b) 250 mg of testosterone enanthate by intramuscular injection in intervals of two to three weeks.

### EXAMPLE 8

*Exemplary treatment of an adult female AIDS patient.* The patient is administered (a) Decapeptyl-Depot® (triptorelin pamoate) 3.75 mg by intramuscular injection in intervals of one month; (b) oestradiol 2 mg daily, *per os;* (c) medroxyprogesterone 5 or 10 mg daily, *per os.*

### EXAMPLE 9

*Pharmaceutical composition of the invention for treating a male adult person.* Triptorelin pamoate (3.75 mg, 10-25 µm powder) was mixed with testosterone enanthate (250 mg, 8-30 µm powder) and suspended in 800 µl of benzyl alcohol:castor oil 2:3 (v/v) for intramuscular injection.

*Pharmaceutical composition kit of the invention for treating an female adult person.* The kit for monthly treatment comprises in one package: (a) Zoladex® for one subcutaneous injection (corresponding to 3.6 mg goserelin); (b) oestradiol tablets (2 mg), once daily *per os;* (c) medroxyprogesterone acetate in depot form (Depo-Provera®) 50 mg, for one intramuscular injection.

### References

1. Williams Textbook of Endocrinology, 10th ed. Larsen P R et al, Eds. Saunders 2002.
2. Gonadotropin-releasing hormone increases CD4-T-lymphocyte numbers in an animal model of immunodeficiency. Jacobson J D et al., J Allergy Clin Immunol. 1999;104:653-8.
3. Immunomodulatory actions of gonadal steroids may be mediated by gonadotropin-releasing hormone. Jacobson J D and Ansari M A, Endocrinology 2004;145(1):330-6.
4. Unusual morphologic features of uterine leiomyomas treated with gonadotropin-releasing hormone agonists: massive lymphoid infiltration and vasculitis. McClean G and McCluggage W G, Int J Surg Pathol. 2003;11(4):339-44.
5. Massive lymphocytic infiltration of uterine leyomyomas associated with GnRH agonist treatment. Bardsley V et al., Histopathology 1998;33(1):80-2.
6. Chronic plasma cell endometritis in hysterectomy specimens of HIV-infected women: a retrospective analysis. Kerr-Layton J A et al., Infect Dis Obstet Gynecol. 1998;6(4):186-90
7. Serum dihydrotestosterone and testosterone concentrations in human immunodeficiency virus-infected men with and without weight loss. Arver S et al., J Androl 1999;20(5):611-8.
8. Prevalence of endocrine dysfunction in HIV-infected men. Brockmeyer G et al., Horm Res 2000;54(5-6):294-5.

## Claims

1. GnRH or GnRH analog including pharmaceutically acceptable salts thereof for use in a method of treating viral disease wherein the method comprises the concomitant administration of GnRH or GnRH analog including pharmaceutically acceptable salts thereof in an amount sufficient to maintain in the patient an elevated unphysiological plasma level of GnRH or GnRH analog,
and of one or several natural, semi-synthetic or synthetic sexual hormones in an amount sufficient to compensate for the castration effect of GnRH or GnRH analog.

2. GnRH or GnRH analog including pharmaceutically acceptable salts thereof for use according to claim 1 wherein the elevated unphysiological plasma level of GnRH or GnRH analog is a castrating plasma level.

3. GnRH or GnRH analog including pharmaceutically acceptable salts thereof for use according to claim 1 or claim 2, wherein the treatment period is for one month or longer.

4. GnRH or GnRH analog including pharmaceutically acceptable salts thereof for use according to claim 1 or claim 2 wherein the treatment period is more than half a year or a year or even longer.

5. GnRH or GnRH analog including pharmaceutically acceptable salts thereof for use according to any of claims 1 to 4, comprising intramuscular, subcutaneous, sublingual or nasal administration.

6. GnRH or GnRH analog including pharmaceutically acceptable salts thereof for use according to any of claims 1 to 5, wherein the viral disease is selected from: Adenovirus Infection, Alphavirus Infection, Arbovirus Encephalitis, Borna Disease, Bunyavirus Infection, Calicivirus Infection, Chickenpox, Condyloma Acuminata, Coronavirus Infection, Coxsackievirus Infection, Cytomegalovirus Infection, Dengue fever, Contageous Ecthyma, Epstein-Barr Virus Infection, Erythema Infectiosum, Hantavirus Infection, Viral Hemorrhagic Fever, Viral Hepatitis, Herpes Simplex, Herpes Zoster, Infectious Mononucleosis, Influenza, Lassa Fever, Measles, Molluscum Contagiosum, Mumps, Paramyxovirus Infection, Phlebotomus Fever, Polyomavirus Infection, Rabies, Respiratory Syncytial Virus Infection, Rift Valley Fever, Rubella, Slow Virus Diseases, Smallpox, Subacute Sclerosing Panencephalitis, Tumor Virus Infections, West Nile Fever, and Yellow Fever.

7. GnRH or GnRH analog including pharmaceutically acceptable salts thereof for use according to any of claims 1 to 5, wherein the viral disease is HIV/AIDS.

8. GnRH or GnRH analog including pharmaceutically acceptable salts thereof for use according to any of claims 1 to 7, wherein the GnRH analog is selected from desorelin, avorelin, leuprolide, triptorelin, buserelin, fertirelin, lutrelin, goserelin, historelin, nafarelin.

9. GnRH or GnRH analog including pharmaceutically acceptable salts thereof for use according to claim 8, wherein the GnRH analog is in the form of a depot formulation for subcutaneous injection.

10. GnRH or GnRH analog including pharmaceutically acceptable salts thereof for use according to claim 9, wherein the GnRH analog is triptorelin acetate or wherein the GnRH analog is goserelin pamoate.

11. GnRH or GnRH analog including pharmaceutically acceptable salts thereof for use according to any of claims 1-10, wherein GnRH or GnRH analog is administered in an amount to cause suppression of FSH and LH release of more than 80 % for a period of one month or longer.

12. GnRH or GnRH analog including pharmaceutically acceptable salts thereof for use according to claim 11, wherein suppression is more than 90 %.

13. GnRH or GnRH analog including pharmaceutically acceptable salts thereof for use according to any of claims 1 to 12, wherein the diseased person is an adult male and wherein the one or several natural, semi-synthetic or synthetic sexual hormone (s) is testosterone or an agent having a corresponding hormonal effect.

14. GnRH or GnRH analog including pharmaceutically acceptable salts thereof for use according to claim 13 wherein the agent having a corresponding hormonal effect is a testosterone analog.

15. GnRH or GnRH analog including pharmaceutically acceptable salts thereof for use according to any of claims 1 to 12, wherein the diseased person is an adult female and wherein the one or several natural, semi-synthetic or synthetic sexual hormone (s) is an oestrogen or a synthetic or semi- synthetic oestrogen analog.

16. GnRH or GnRH analog including pharmaceutically acceptable salts thereof for use according to claim 15 wherein the oestrogen is oestradiol or a semi-synthetic ester of oestradiol.

17. GnRH or GnRH analog including pharmaceutically acceptable salts thereof for use according to claim 15 or 16, comprising the administration of a gestagen.

18. A composition for use in the treatment of viral disease comprising GnRH or a GnRH analog, one or several natural, semi-synthetic or synthetic sexual hormones, and a pharmaceutically acceptable carrier, wherein the GnRH or GnRH analog is present in an amount sufficient to maintain in a patient an elevated unphysiological plasma level of GnRH or GnRH analog and wherein the one or several natural, semi-synthetic or synthetic sexual hormones is/are present in an amount sufficient to compensate for the castration effect of GnRH or GnRH analog.

19. The composition for use according to claim 18 wherein the unphysiological amount of GnRH or GnRH analog is a castrating amount.

20. The composition for use according to claim 18 or claim 19 wherein the diseased person is an adult male and the one or several natural, semi-synthetic or synthetic sexual hormones is selected from testosterone or an agent exhibiting a corresponding hormonal effect.

21. The composition for use according to claim 18 or claim 19 wherein the diseased person is an adult female and the one or several natural, semi-synthetic or synthetic sexual hormones is selected from oestradiol or an agent exhibiting a corresponding hormonal effect.

22. The composition for use according to claim 21 wherein the composition additionally comprises a progestagen.

23. The composition for use according to any of claims 18 to 22 in the form of a depot or slow or sustained release composition.

24. The composition for use according to claim 23, comprising a bioerodible matrix.

## Patentansprüche

1. GnRH oder GnRH-Analogon, einschließlich pharmazeutisch annehmbarer Salze davon, zur Verwendung in einem Verfahren zur Behandlung viraler Erkrankung, wobei das Verfahren die gleichzeitige Verabreichung umfasst von
GnRH oder GnRH-Analogon, einschließlich pharmazeutisch annehmbarer Salze davon, in einer Menge, die ausreichend ist, im Patienten einen erhöhten unphysiologischen Plasmaspiegel von GnRH oder GnRH-Analogon aufrechtzuerhalten,
und von einem oder mehreren natürlichen, semi-synthetischen oder synthetischen Sexualhormonen in einer Menge, die ausreichend ist, den Kastrationseffekt von GnRH oder GnRH-Analogon zu kompensieren.

2. GnRH oder GnRH-Analogon, einschließlich pharmazeutisch annehmbarer Salze davon, zur Verwendung nach Anspruch 1, wobei der erhöhte unphysiologische Plasmaspiegel von GnRH oder GnRH-Analogon ein kastrierender Plasmaspiegel ist.

3. GnRH oder GnRH-Analogon, einschließlich pharmazeutisch annehmbarer Salze davon, zur Verwendung nach Anspruch 1 oder Anspruch 2, wobei der Behandlungszeitraum für einen Monat oder länger ist.

4. GnRH oder GnRH-Analogon, einschließlich pharmazeutisch annehmbarer Salze davon, zur Verwendung nach Anspruch 1 oder Anspruch 2, wobei der Behandlungszeitraum mehr als ein halbes Jahr oder ein Jahr oder sogar länger ist.

5. GnRH oder GnRH-Analogon, einschließlich pharmazeutisch annehmbarer Salze davon, zur Verwendung nach einem der Ansprüche 1 bis 4, umfassend intramuskuläre, subkutante, sublinguale oder nasale Verabreichung.

6. GnRH oder GnRH-Analogon, einschließlich pharmazeutisch annehmbarer Salze davon, zur Verwendung nach einem der Ansprüche 1 bis 5, wobei die virale Erkrankung ausgewählt ist aus:
Adenovirus-Infektion, Alphavirus-Infektion, Arbovirus-Enzephalitis, Borna-Krankheit, Bunyavirus-Infektion, Calicivirus-Infektion, Windpocken, Condyloma acuminata, Coronavirus-Infektion, Coxsackievirus-Infektion, Zytomegalievirus-Infektion, Dengue-Fieber, Ecthyma contagiosum, Epstein-Barr-Virus-Infektion, Erythema infectiosum, Hantavirus-Infektion, virales Hämorrhagisches Fieber, virale Hepatitis, Herpes simplex, Herpes Zoster, infektiöse Mononukleose, Influenza, Lassa-Fieber, Masern, Molluscum contagiosum, Mumps, Paramyxovirus-Infektion, Phlebotomus-Fieber, Polyomavirus-Infektion, Tollwut, Respiratorisches-Synzytial-Virus-Infektion, Rifttal-Fieber, Röteln, Langsame-Virus-Krankheiten, Pocken, subakute sklerosierende Panenzephalitis, Tumor-Virus-Infektionen, West-Nil-Fieber und Gelbfieber.

7. GnRH oder GnRH-Analogon, einschließlich pharmazeutisch annehmbarer Salze davon, zur Verwendung nach einem der Ansprüche 1 bis 5, wobei die virale Erkrankung HIV/AIDS ist.

8. GnRH oder GnRH-Analogon, einschließlich pharmazeutisch annehmbarer Salze davon, zur Verwendung nach einem der Ansprüche 1 bis 7, wobei das GnRH-Analogon ausgewählt ist aus Desorelin, Avorelin, Leuprolid, Triptorelin, Buserelin, Fertirelin, Lutrelin, Goserelin, Historelin, Nafarelin.

9. GnRH oder GnRH-Analogon, einschließlich pharmazeutisch annehmbarer Salze davon, zur Verwendung nach Anspruch 8, wobei das GnRH-Analogon in Form einer Depot-Formulierung für subkutane Injektion vorliegt.

10. GnRH oder GnRH-Analogon, einschließlich pharmazeutisch annehmbarer Salze davon, zur Verwendung nach Anspruch 9, wobei das GnRH-Analogon Triptorelinacetat ist, oder wobei das GnRH-Analogon Goserelinpamoat ist.

11. GnRH oder GnRH-Analogon, einschließlich pharmazeutisch annehmbarer Salze davon, zur Verwendung nach einem der Ansprüche 1-10, wobei GnRH oder GnRH-Analogon verabreicht wird in einer Menge, um Unterdrückung von FSH- und LH-Freisetzung von mehr als 80 % für einen Zeitraum von einem Monat oder länger zu bewirken.

12. GnRH oder GnRH-Analogon, einschließlich pharmazeutisch annehmbarer Salze davon, zur Verwendung nach Anspruch 11, wobei Unterdrückung mehr als 90 % ist.

13. GnRH oder GnRH-Analogon, einschließlich pharmazeutisch annehmbarer Salze davon, zur Verwendung nach einem der Ansprüche 1 bis 12, wobei die erkrankte Person ein erwachsener Mann ist, und wobei das eine oder die mehreren natürlichen, semi-synthetischen oder synthetischen Sexualhormon(e) Testosteron oder ein Mittel mit einem entsprechenden hormonellen Effekt ist.

14. GnRH oder GnRH-Analogon, einschließlich pharmazeutisch annehmbarer Salze davon, zur Verwendung nach Anspruch 13, wobei das Mittel mit einem entsprechenden hormonellen Effekt ein Testosteron-Analogon ist.

15. GnRH oder GnRH-Analogon, einschließlich pharmazeutisch annehmbarer Salze davon, zur Verwendung nach einem der Ansprüche 1 bis 12, wobei die erkrankte Person eine erwachsene Frau ist, und wobei das eine oder die mehreren natürlichen, semi-synthetischen oder synthetischen Sexualhormon(e) ein Estrogen oder ein synthetisches oder semi-synthetisches Estrogen-Analogon ist.

16. GnRH oder GnRH-Analogon, einschließlich pharmazeutisch annehmbarer Salze davon, zur Verwendung nach Anspruch 15, wobei das Estrogen Estradiol oder ein semi-synthetischer Ester von Estradiol ist.

17. GnRH oder GnRH-Analogon, einschließlich pharmazeutisch annehmbarer Salze davon, zur Verwendung nach Anspruch 15 oder 16, umfassend die Verabreichung eines Gestagens.

18. Zusammensetzung zur Verwendung in Behandlung viraler Erkrankung, umfassend GnRH oder ein GnRH-Analogon, eines oder mehrere natürliche oder semi-synthetische oder synthetische Sexualhormone und einen pharmazeutisch annehmbaren Träger, wobei das GnRH oder GnRH-Analogon in einer Menge vorhanden ist, die ausreichend ist, in einem Patienten einen erhöhten unphysiologischen Plasmaspiegel von GnRH oder GnRH-Analogon aufrechtzuerhalten, und wobei das eine oder die mehreren natürlichen, semi-synthetischen oder synthetischen Sexualhormone in einer Menge vorhanden ist/sind, die ausreichend ist, den Kastrationseffekt von GnRH oder GnRH-Analogon zu kompensieren.

19. Zusammensetzung zur Verwendung nach Anspruch 18, wobei die unphysiologische Menge von GnRH oder GnRH-Analogon eine kastrierende Menge ist.

20. Zusammensetzung zur Verwendung nach Anspruch 18 oder Anspruch 19, wobei die erkrankte Person ein erwachsener Mann ist und das eine oder die mehreren natürlichen, semi-synthetischen oder synthetischen Sexualhormone ausgewählt ist aus Testosteron oder einem Mittel, welches einen entsprechenden hormonellen Effekt aufweist.

21. Zusammensetzung zur Verwendung nach Anspruch 18 oder Anspruch 19, wobei die erkrankte Person eine erwachsene Frau ist und das eine oder die mehreren natürlichen, semi-synthetischen oder synthetischen Sexualhormone ausgewählt ist aus Estradiol oder einem Mittel, welches einen entsprechenden hormonellen Effekt aufweist.

22. Zusammensetzung zur Verwendung nach Anspruch 21, wobei die Zusammensetzung zusätzlich ein Progestagen umfasst.

23. Zusammensetzung zur Verwendung nach einem der Ansprüche 18 bis 22 in Form einer Depotzusammensetzung oder einer Zusammensetzung mit langsamer oder kontinuierlicher Freisetzung.

24. Zusammensetzung zur Verwendung nach Anspruch 23, umfassend eine bioerodierbare Matrix.

## Revendications

1. GnRH ou analogue de la GnRH y compris leurs sels pharmaceutiquement acceptables pour une utilisation dans une méthode de traitement d'une maladie virale, la méthode comprenant l'administration simultanée de GnRH ou d'un analogue de la GnRH y compris de leurs sels pharmaceutiquement acceptables dans une quantité suffisante pour maintenir chez le patient un taux plasmatique non physiologique élevé de GnRH ou d'analogue de la GnRH,
et d'une ou de plusieurs hormones sexuelles naturelles, semi-synthétiques ou synthétiques dans une quantité suffisante pour compenser l'effet de castration de la GnRH ou de l'analogue de la GnRH.

2. GnRH ou analogue de la GnRH y compris leurs sels pharmaceutiquement acceptables pour une utilisation selon la revendication 1, où le taux plasmatique non physiologique élevé de la GnRH ou de l'analogue de la GnRH est un taux plasmatique castrateur.

3. GnRH ou analogue de la GnRH y compris leurs sels pharmaceutiquement acceptables pour une utilisation selon la revendication 1 ou la revendication 2, où la période de traitement est d'un mois ou plus longue.

4. GnRH ou analogue de la GnRH y compris leurs sels pharmaceutiquement acceptables pour une utilisation selon la revendication 1 ou la revendication 2, où la période de traitement est supérieure à un demi-an ou un an ou même plus longue.

5. GnRH ou analogue de la GnRH y compris leurs sels pharmaceutiquement acceptables pour une utilisation selon l'une quelconque des revendications 1 à 4, comprenant une administration intramusculaire, sous-cutanée, sublinguale ou nasale.

6. GnRH ou analogue de la GnRH y compris leurs sels pharmaceutiquement acceptables pour une utilisation selon l'une quelconque des revendications 1 à 5, où la maladie virale est choisie parmi : une infection par un adénovirus, une infection par un alphavirus, une encéphalite due à un arbovirus, la maladie de Borna, une infection par un bunyavirus, une infection par un calicivirus, la varicelle, les condylomes acuminés, une infection par un coronavirus, une infection par un virus Coxsackie, une infection par un cytomégalovirus, la fièvre dengue, l'ecthyma contagieux, une infection par le virus d'Epstein Barr, un érythème infectieux, une infection par un hantavirus, une fièvre hémorragique virale, une hépatite virale, un herpès simplex, le zona, la mononucléose infectieuse, la grippe, la fièvre de Lassa, la rougeole, le molluscum contagiosum, les oreillons, une infection par un paramyxovirus, la fièvre à phlébotomes, une infection par un polyomavirus, la rage, une infection par le virus respiratoire syncytial, la fièvre de la vallée du Rift, la rubéole, les maladies virales lentes, la variole, la panencéphalite sclérosante subaiguë, les infections par des virus tumoraux, la fièvre du Nil occidental, et la fièvre jaune.

7. GnRH ou analogue de la GnRH y compris leurs sels pharmaceutiquement acceptables pour une utilisation selon l'une quelconque des revendications 1 à 5, où la maladie virale est le VIH/SIDA.

8. GnRH ou analogue de la GnRH y compris leurs sels pharmaceutiquement acceptables pour une utilisation selon l'une quelconque des revendications 1 à 7, où l'analogue de la GnRH est choisi parmi la désoréline, l'avoréline, le leuprolide, la triptoréline, la buséréline, la fertiréline, la lutréline, la goséréline, l'historéline, la nafaréline.

9. GnRH ou analogue de la GnRH y compris leurs sels pharmaceutiquement acceptables pour une utilisation selon la revendication 8, où l'analogue de la GnRH est sous la forme d'une formulation retard pour une injection sous-cutanée.

10. GnRH ou analogue de la GnRH y compris leurs sels pharmaceutiquement acceptables pour une utilisation selon la revendication 9, où l'analogue de la GnRH est l'acétate de triptoréline ou bien où l'analogue de la GnRH est le pamoate de goséréline.

11. GnRH ou analogue de la GnRH y compris leurs sels pharmaceutiquement acceptables pour une utilisation selon l'une quelconque des revendications 1 à 10, où la GnRH ou l'analogue de la GnRH est administré dans une quantité pour provoquer une suppression de la libération de FSH et de LH de plus de 80 % pendant une période d'un mois ou plus longue.

12. GnRH ou analogue de la GnRH y compris leurs sels pharmaceutiquement acceptables pour une utilisation selon la revendication 11, où la suppression est de plus de 90 %.

13. GnRH ou analogue de la GnRH y compris leurs sels pharmaceutiquement acceptables pour une utilisation selon l'une quelconque des revendications 1 à 12, où la personne malade est un homme adulte et où les une ou plusieurs hormones sexuelles naturelles, semi-synthétiques ou synthétiques sont la testostérone ou un agent ayant un effet hormonal correspondant.

14. GnRH ou analogue de la GnRH y compris leurs sels pharmaceutiquement acceptables pour une utilisation selon la revendication 13, où l'agent ayant un effet hormonal correspondant est un analogue de la testostérone.

15. GnRH ou analogue de la GnRH y compris leurs sels pharmaceutiquement acceptables pour une utilisation selon l'une quelconque des revendications 1 à 12, où la personne malade est une femme adulte et où les une ou plusieurs hormones sexuelles naturelles, semi-synthétiques ou synthétiques sont un oestrogène ou un analogue d'oestrogène synthétique ou semi-synthétique.

16. GnRH ou analogue de la GnRH y compris leurs sels pharmaceutiquement acceptables pour une utilisation selon la revendication 15, où l'oestrogène est l'oestradiol ou un ester semi-synthétique de l'oestradiol.

17. GnRH ou analogue de la GnRH y compris leurs sels pharmaceutiquement acceptables pour une utilisation selon la revendication 15 ou 16, comprenant l'administration d'un gestagène.

18. Composition pour une utilisation dans le traitement d'une maladie virale comprenant de la GnRH ou un analogue de la GnRH, une ou plusieurs hormones sexuelles naturelles, semi-synthétiques ou synthétiques, et un support pharmaceutiquement acceptable, dans laquelle la GnRH ou l'analogue de la GnRH est présent dans une quantité suffisante pour maintenir chez un patient un taux plasmatique non physiologique élevé de GnRH ou d'analogue de la GnRH et où les une ou plusieurs hormones sexuelles naturelles, semi-synthétiques ou synthétiques est/sont présentes dans une quantité suffisante pour compenser l'effet de castration de la GnRH ou de l'analogue de la GnRH.

19. Composition pour une utilisation selon la revendication 18, où la quantité non physiologique de GnRH ou d'analogue de la GnRH est une quantité castratrice.

20. Composition pour une utilisation selon la revendication 18 ou la revendication 19, où la personne malade est un homme adulte et les une ou plusieurs hormones sexuelles naturelles, semi-synthétiques ou synthétiques sont choisies parmi la testostérone ou un agent présentant un effet hormonal correspondant.

21. Composition pour une utilisation selon la revendication 18 ou la revendication 19, où la personne malade est une femme adulte et les une ou plusieurs hormones sexuelles naturelles, semi-synthétiques ou synthétiques sont choisies parmi l'oestradiol ou un agent présentant un effet hormonal correspondant.

22. Composition pour une utilisation selon la revendication 21, où la composition comprend en outre un progestagène.

23. Composition pour une utilisation selon l'une quelconque des revendications 18 à 22, sous la forme d'une composition retard ou à libération lente ou prolongée.

24. Composition pour une utilisation selon la revendication 23, comprenant une matrice bioérodable.
